(19) Europäisches Patentamt
European Patent Office
Office européen des brevets

(11) Veröffentlichungsnummer: **0 170 951**
**A1**

(12) # EUROPÄISCHE PATENTANMELDUNG

(21) Anmeldenummer: **85109007.6**

(22) Anmeldetag: **18.07.85**

(51) Int. Cl.4: **C 07 C 107/04**, C 07 C 121/82 // A61K31/655

(30) Priorität: **19.07.84 DE 3426644**

(43) Veröffentlichungstag der Anmeldung: **12.02.86 Patentblatt 86/7**

(84) Benannte Vertragsstaaten: **AT BE CH DE FR GB IT LI NL SE**

(71) Anmelder: **Deutsches Krebsforschungszentrum, Im Neuenheimer Feld 280, D-6900 Heidelberg 1 (DE)**

(72) Erfinder: **Kòlar, George, Dr., Lattweg 13, D-6902 Sandhausen (DE)**
Erfinder: **Schendzielorz, Michael, Siebseeweg 3a, D-6800 Mannheim (DE)**

(74) Vertreter: **Patentanwälte Müller-Boré, Deufel, Schön, Hertel, Lewald, Otto, Postfach 26 02 47 Isartorplatz 6, D-8000 München 26 (DE)**

(54) **N,N-bis-(1-Aryl-3-methyltriazen-3-yl)methylether.**

(57) Die Erfindung betrifft neue N,N-bis[(1-Aryl-3-methyltriazen-3-yl)methyl]ether der allgemeinen Formel

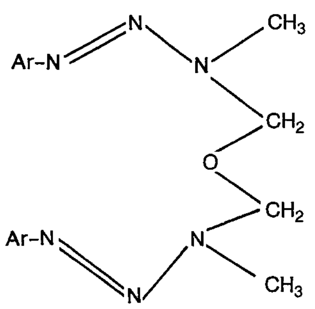

I

worin Ar eine durch einen elektronenziehenden Rest substituierte Arylgruppe ist, sowie ein Verfahren zur Herstellung dieser Ether durch Umsetzung eines entsprechend substituierten Aryl-3-methyl-3-hydroxymethyltriazens mit einer äquimolaren Menge Tosylchlorid in Gegenwart einer Stickstoffbase. Diese neuen Ether sind wertvolle Chemotherapeutika, insbesondere eignen sie sich als Immunsystemstimulantien oder Zytostatika.

EP 0 170 951 A1

N-N-bis-⌊(1-Aryl-3-methyltriazen-3-yl)methyl⌋ether.

Die Erfindung betrifft neue N,N-bis-⌊(1-Aryl-3-methyltriazen-3yl)methyl⌋ether der folgenden Fomel

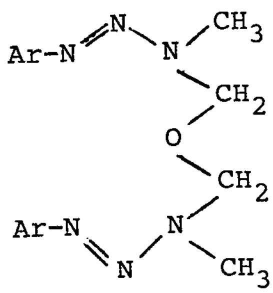

I

worin Ar eine durch einen elektronenziehenden Rest substituierte Arylgruppe ist.

Diese Verbindungen eignen sich als Chemoterapeutika, insbesondere als Immunsystemstimulantien oder Zytostatika.

Besonders bevorzugt sind diejenigen Verbindungen, in denen Ar für eine substituierte Phenylgruppe steht, insbesondere für eine in der p-Stellung substituierte Phenylgruppe, d.h., daß es sich um Verbindungen der Formel

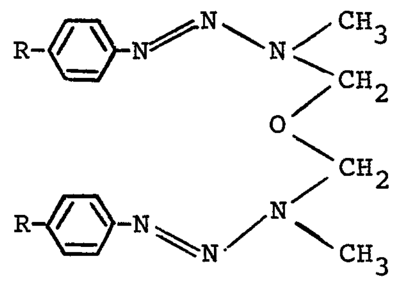

III

handelt.

Die an der Arylgruppe sitzenden elektronenziehenden Gruppen besitzen vorzugsweise eine Hammet-Konstante $\sigma$ von 0,20 bis 0,80. Es handelt sich insbesondere um gegebenenfalls verzweigte halogenierte Niedrigalkylgruppen mit 1 bis 6 Kohlenstoffatomen, halogenierte Cycloalkylgruppen mit bis zu 8 Kohlenstoffatomen, dem Rest $COOR_1$ , worin $R_1$ eine Niedrigalkylgruppe mit 1 bis 3 Kohlenstoffatomen bedeutet oder um -CN oder $-NO_2$. Besonders bevorzugt werden dabei die folgenden Gruppen: -CN, $-NO_2$, $-COOCH_3$, $-COOCH_2CH_3$ und $CF_3$.

Die Herstellung der neuen Ether erfolgt in der Weise, daß ein durch eine elektronenziehende Gruppe substituiertes Aryl-3-methyl-3-hydroxymethyl-triazen der folgenden Formel

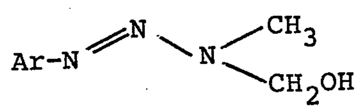

II

mit einer äquimolaren Menge Tosylchlorid in einer Stickstoffbase, wie Triethylamin oder Pyridin, zur Umsetzung gebracht wird. Vorzugsweise erfolgt die Umsetzung in Gegenwart eines Lösungsmittels, insbesondere eines

chlorierten Kohlenwasserstoffs, wie Trichlormethan ($CHCl_3$).

Zweckmäßig wird das erfindungsgemäße Verfahren in der Weise durchgeführt, daß eine Lösung des substituierten Aryl-3-methyl-3-hydroxymethyltriazens in dem wasserfreien Lösungsmittel aufgelöst wird. Zu dieser Lösung wird dann beispielsweise Triethylamin hinzugegeben, worauf das Tosylchlorid, ebenfalls in Lösung in einem Lösungsmittel, der ersten Lösung langsam zugetropft wird. Nach beendeter Zugabe wird noch einige Zeit, vorzugsweise 1 Stunde, gerührt und dann anschließend zum Rückfluß, vorzugsweise während 2 Stunden erhitzt. Nach der Entfernung des Lösungsmittels im Vakuum wird das Produkt mehrmals mit Wasser gewaschen und wieder in dem Lösungsmittel aufgelöst, worauf nochmals mit Wasser gewaschen wird. Die organische Phase wird über Natriumsulfat getrocknet.Nachdem das Lösungsmittel im Vakuum entfernt worden ist, wird das erhaltene ölige Rohprodukt mit einem entsprechenden Lösungsmittel aufgenommen, worauf es kristallin ausfällt. Es wird dann nochmals aus dem entsprechenden Lösungsmittel umkristallisiert.

Die Herstellung der als Ausgangsmaterialien eingesetzten substituierten Aryl-3-methyl-3-hydroxymethyltriazene der Formel II, bei denen es sich um bekannte Verbindungen handelt, erfolgt in zweckmäßiger Weise nach der Methode von Keith Vaugham "Journal of Medicinal Chemistry", 1984, Band 27, Nr. 3, Seiten 357 ff. oder nach der Arbeitsweise, wie sie in "Chemical Carcinogens", American Chemical Society Monograph Series No. 173, Washington D.C., beschrieben wird.

Die erfindungsgemäßen Produkte sind kristalline Substanzen mit definierten Schmelzpunkten.

Beispiel

In der nachfolgend beschriebenen Weise werden erfindungsgemäße Verbindungen hergestellt, in denen Ar für eine p-Stellung durch $-COOCH_3$ bzw.eine in p-Stellung substituierte -CN-Gruppe steht.

0,01 Mol des jeweiligen substituierten 1-Aryl-3-methylhydroxymethyltriazens werden in 50 ml absolutem $CHCL_3$ gelöst. Zu dieser Lösung gibt man 1,21 g (0,012 Mol) Triethylamin. 1,90 g (0,01 Mol) Tosylchlorid werden in 10 ml $CHCl_3$ aufgelöst, worauf diese Lösung langsam der ersten Lösung zugetropft wird. Nach beendeter Zugabe wird noch 1 Stunde gerührt und anschließend 2 Stunden zum Rückfluß erhitzt.

Nachdem das Lösungsmittel im Vakuum entfernt worden ist, wird das Produkt mehrmals mit Wasser ausgewaschen, worauf das Rohprodukt wieder in $CHCl_3$ aufgenommen wird. Dann wird nochmals mit Wasser gewaschen. Die organische Phase wird über Natriumsulfat getrocknet. Nachdem das Lösungsmittel im Vakuum entfernt worden ist, wird das ölige Produkt in einem geeigneten Lösungsmittel aufgenommen, worauf es kristallin ausfällt. Es wird dann nochmals aus dem entsprechenden Lösungsmitel umkristallisiert.

Die dabei erhaltenen Produkte besitzen folgende Schmelzpunkte:

1. Produkt,dessen Phenylgruppe in der p-Stellung mit $-COOCH_3$ substituiert ist: 168 bis 169°C.

2. Produkt, dessen Phenylgruppe in der p-Stellung mit -CN substituiert ist: 203 bis 204°C.

**Patentanwälte · European Patent Attorneys**

Dr. W. Müller-Boré †

Dr. Paul Deufel
Dipl.-Chem., Dipl.-Wirtsch.-Ing.

Dr. Alfred Schön
Dipl.-Chem.

Werner Hertel
Dipl.-Phys.

Dietrich Lewald
Dipl.-Ing.

Dr.-Ing. Dieter Otto
Dipl.-Ing.

Dr. Müller-Boré und Partner · POB 260247 · D-8000 München 26

K 1702

Deutsches Krebsforschungszentrum
Stiftung des öffentlichen Rechts
Im Neuenheimer Feld 280
6900 Heidelberg 1

N,N-bis-[(1-Aryl-3-methyltriazen-3-yl)methyl]ether

## Patenansprüche

1. N,N-bis[(1-Aryl-3-methyltriazen-3-yl)methyl]ether der allgemeinen Formel

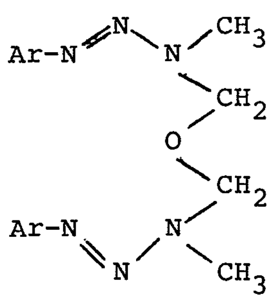

I

worin Ar eine durch einen elektronenziehenden Rest substituierte Arylgruppe ist.

2. Ether nach Anspruch 1, dadurch gekennzeichnet, daß Ar für eine substituierte Phenylgruppe steht.

3. Ether nach Anspruch 2, dadurch gekennzeichnet, daß die Phenylgruppen in der p-Stellung substituiert sind.

4. Ether nach den Ansprüchen 1 bis 3, dadurch gekennzeichnet, daß die elektronenziehenden Gruppen eine Hammet-Konstante $\sigma$ von 0,20 bis 0,80 besitzen .

5. Ether nach Anspruch 4, dadurch gekennzeichnet, daß die elektronenziehenden Gruppen aus einer gegebenenfalls verzweigten halogenierten Niedrigalkylgruppe mit 1 bis 6 Kohlenstoffatomen, einer halogenierten Cycloalkylgruppe mit bis zu 8 Kohlenstoffatomen, dem Rest $COOR_1$, worin $R_1$ eine Niedrigalkylgruppe mit 1 bis 3 Kohlenstoffatomen bedeutet, oder aus CN oder $NO_2$ besteht.

6. Verfahren zur Herstellung von N,N-bis-[(1-Aryl-3-methyltriazen-3-yl)methyl]ethern gemäß Anspruch 1, dadurch gekennzeichnet, daß ein mit einer elektronenziehenden Gruppe substituiertes Aryl-3-methyl-3-hydroxymethyltriazen der Formel

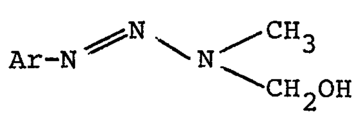

II

mit einer äquimolaren Menge Tosylchlorid in Gegenwart einer Stickstoffbase zur Umsetzung gebracht wird.

7. Verfahren nach Anspruch 6, dadurch gekennzeichnet, daß die Umsetzung in Gegenwart eines Lösungsmittels, insbesondere eines chlorierten Lösungsmittels, durchgeführt wird.

8. Verfahren nach den Ansprüchen 6 bis 7, dadurch gekennzeichnet, daß das Hydroxymethyltriazen und die Stickstoffbase in einem Lösungsmittel vorgelegt werden und anschließend das Tosylchlorid in Lösung in einem Lösungsmittel zugetropft wird.

9. Verfahren nach Anspruch 8, dadurch gekennzeichnet, daß nach erfolgter Zugabe des Tosylchlorids die Reaktionsmischung gerührt, zum Rückfluß erhitzt, das Lösungsmittel im Vakuum entfernt und der gebildete Ether in üblicher Weise gewonnen wird.

Europäisches Patentamt

0170951

Nummer der Anmeldung

EP 85 10 9007

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| Y | JOURNAL OF THE CHEMICAL SOCIETY, CHEMICAL COMMUNICATIONS, Nr. 13, 1983, Seiten 721-722, US; R.J. LAFRANCE et al.: "N,N-bis-(1-aryl-3-methyltriazen-3-ylmethyl)methylamines(1,9-diaryl-3,5,7-trimethyl-1,2,3,5,7,8,9-hepta-azanona-1,8-dienes): novel coupling products from the reaction of arenediazonium ions with methylamine and formaldehyde" * Seiten 721-722 * | 1 | C 07 C 107/04<br>C 07 C 121/82 //<br>A 61 K 31/655 |
| | --- | | |
| Y | CANADIAN JOURNAL OF CHEMISTRY, Band 62, Nr. 3, 1984, Seiten 741-749, US; C.M. HEMENS et al.: "Open-chain nitrogen compounds. Part V. Hydroxymethyltriazenes: synthesis of some new alkyl homologues of the anti-tumour 3-methyl-3-hydroxymethyltriazenes and preparation of the derived acetoxymethyl-, benzoyloxymethyl-, and methoxymethyltriazenes" * Seiten 741-742 * | 1 | RECHERCHIERTE SACHGEBIETE (Int. Cl. 4)<br>C 07 C 107/00<br>C 07 C 121/00<br>A 61 K 31/00 |
| | ----- | | |

Der vorliegende Recherchenbericht wurde für alle Patentansprüche erstellt.

| Recherchenort | Abschlußdatum der Recherche | Prüfer |
|---|---|---|
| DEN HAAG | 25-10-1985 | MOREAU J.M. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82